# EUROPÄISCHE PATENTSCHRIFT

(11) **EP 2 907 527 B1**
(45) Veröffentlichungstag und Bekanntmachung des Hinweises auf die Patenterteilung: **24.08.2016**
(21) Anmeldenummer: 15154264.4
(22) Anmeldetag: 09.02.2015
(51) Int. Cl.: A61L 2/10, B65B 55/16

(54) **Betriebsverfahren für eine Bestrahlungsvorrichtung**
Operating method for an irradiation device
Procédé de fonctionnement pour un dispositif de rayonnement

(30) Priorität: 17.02.2014 DE 102014101935
(43) Veröffentlichungstag der Anmeldung: 19.08.2015
(73) Patentinhaber: Heraeus Noblelight GmbH, 63450 Hanau (DE)
(72) Erfinder: Lott, Josef Zoltan, 22763 Hamburg (DE); Brieden, Karl-Wilhelm, 63579 Freigericht (DE); Schloemp, Silke, 63571 Gelnhausen (DE)
(74) Vertreter: Heraeus IP

(56) Entgegenhaltungen:
- WO-A1-2010/063720
- JP-A- 2008 265 830
- US-A1- 2004 140 435
- US-B1- 6 563 255

## Beschreibung

### Technisches Gebiet

Die vorliegende Erfindung betrifft ein Bestrahlungsvorrichtung zur Bestrahlung eines Substrats mit einem UV-Strahler, umfassend die Verfahrensschritte:
(a) Betreiben des UV-Strahlers mit einer von einer Soll-Betriebstemperatur abhängigen Soll-Betriebsstrahlungsleistung,
(b) kontinuierliches Zuführen des Substrats in einen durch den UV-Strahler festgelegten Bestrahlungsbereich mit einer Zuführgeschwindigkeit,
(c) Bestrahlen des Substrats im Bestrahlungsbereich.

Derartige Betriebsverfahren werden häufig zum Betrieb von Bestrahlungsvorrichtungen in der Fließfertigung eingesetzt, beispielsweise zur Desinfektion, Wasseraufbereitung oder zum Aushärten von Lacken, Kleb- oder Kunststoffen.

### Stand der Technik

Bei bekannten Bestrahlungsvorrichtungen sind als Strahlungsquelle ein oder mehrere UV-Strahler vorgesehen. UV-Strahler in diesem Sinne sind beispielsweise Quecksilberdampf-Niederdruckstrahler, -Mitteldruckstrahler oder Hochdruckstrah-ler. In diesen Bestrahlungsvorrichtungen sind der oder die UV-Strahler derart angeordnet, dass sie einen Bestrahlungsbereich festlegen, innerhalb dessen eine Bestrahlung des Substrats mit einer vorgegebenen Mindestbestrahlungsstärke erfolgt. Das Substrat wird dem Bestrahlungsbereich von einer Transportvorrichtung zugeführt, wobei es den Bestrahlungsbereich möglichst mit einer konstanten Geschwindigkeit durchläuft.

Eine Bestrahlungsvorrichtung dieser Art ist beispielsweise aus JP 2008-265830 A bekannt, wobei Folienbeutel eine UV-Bestrahlungseinrichtung kontinuierlich durchlaufen und dabei sterilisiert werden bevor sie anschließen mit einer Sterilisierflüssigkeit befüllt werden.

Bei gegebener Bestrahlungsleistung der UV-Lampe legt die Verweildauer des Substrats innerhalb des Bestrahlungsbereichs die auf das Substrat auftreffende Bestrahlungsenergie fest. Über eine Regelung der Transportgeschwindigkeit des Substrats kann die auf das Substrat auftreffende Bestrahlungsenergie an den jeweils stattfindenden Bestrahlungsvorgang angepasst werden.

Zur Erzielung einer guten Energieeffizienz ist grundsätzlich ein möglichst kontinuierlicher Betrieb der Bestrahlungsvorrichtung, das heißt ein Betrieb ohne Unterbrechungen wünschenswert. Kommt es zu einer Störung im Fertigungsprozess muss sichergestellt sein, dass ein im Bestrahlungsbereich verbliebenes Substrat nicht durch übermäßige Bestrahlung beschädigt wird.

Um eine Beschädigung des Substrats zu vermeiden, können die UV-Strahler bei einer Unterbrechung des Fertigungsprozesses zwar ausgeschaltet werden. Sie benötigen allerdings bei jedem Einschalten eine gewisse Zeit, um ihre nominelle Strahlungsleistung wieder zu erreichen. Die Strahlungsleistung der UV-Strahler hängt dabei insbesondere von deren Temperatur ab. Bei einem Kaltstart des UV-Strahlers erwärmt sich dieser nach dem Einschalten kontinuierlich bis er seine Betriebstemperatur erreicht. Erst mit dem Erreichen der Betriebstemperatur wird eine konstante Strahlungsleistung erzielt. Die Zeit bis zum Erreichen der Betriebstemperatur wird als Aufheizzeit bezeichnet. Sie beträgt in der Regel mehrere Minuten. Ein Neustart der UV-Lampe geht daher regelmäßig mit einer Verzögerung des Fertigungsprozesses einher.

Um eine möglichst kurze Aufheizzeit nach einer Unterbrechung zu gewährleisten, wird daher im Stand der Technik auf ein Abschalten der UV-Strahler verzichtet. Stattdessen wird der Einsatz eines Abschirmelements zur Unterbrechung des Strahlengangs zwischen UV-Strahler und Substrat vorgeschlagen, so dass der UV-Strahler auch bei einem Stillstand des Fertigungsprozesses weiterbetrieben werden kann, ohne dass er auf das Substrat unmittelbar einwirkt.

Eine solche Bestrahlungsvorrichtung ist aus der JP 06 056 132 A bekannt. Die Bestrahlungsvorrichtung umfasst eine Entkeimungslampe, die einen Bestrahlungsbereich festlegt, sowie eine Transportvorrichtung, die das Substrat durch den Bestrahlungsbereich transportiert. Um bei einem Stillstand der Transportvorrichtung eine übermäßige Bestrahlung eines im Bestrahlungsbereich verbliebenen Substrats zu vermeiden, wird vorgeschlagen, zwischen der UV-Entkeimungslampe und dem Substrat eine Verschlussklappe (Shutter) anzuordnen, die bei einem Stillstand des Fertigungsprozesses den Strahlengang zwischen UV-Entkeimungslampe und Substrat unterbricht.

Die Verschlussklappe hat allerdings den Nachteil, dass sie vom UV-Strahler emittierte Strahlung teilweise absorbiert, teilweise reflektiert, so dass diese ihrerseits zu einer starken lokalen Erwärmung der Umgebung des UV-Strahlers und mithin zu einer Erwärmung des UV-Strahlers beitragen kann. Eine zu starke Erwärmung des UV-Strahlers kann einerseits dessen Strahlungsleistung beeinträchtigen; sie trägt darüber hinaus zu einer Alterung des Strahlers bei, wobei dessen Emission im UV-Bereich sinkt und sich die Lebensdauer des Strahlers verringert.

Darüber hinaus geht ein andauernder Betrieb des UV-Strahlers bei einem längeren Stillstand, mit einem Verbrauch von Energie und häufig auch mit einer Schädigung des zu behandelnden Substrats einher.

Weiterhin setzt der Einsatz einer Verschlussklappe das Vorhandensein eines gewissen Bauraums, also einen ausreichenden Abstand des Strahlers zum Substrat, voraus. Dieser Abstand vermindert allerdings die Bestrahlungsstärke. Grundsätzlich gilt, dass eine möglichst große Bestrahlungsstärke dann erzielt wird, wenn der Abstand zwischen Strahler und Substrat möglichst gering ist.

Schließlich ist eine Verschlusskappe ein bewegliches Bauteil, das angesteuert werden muss und eine gewisse Fehleranfälligkeit aufweist.

### Technische Aufgabe

Der Erfindung liegt daher die Aufgabe zugrunde, ein einfaches und kostengünstiges Betriebsverfahren für eine Bestrahlungsvorrichtung anzugeben, das die oben genannten Nachteile vermeidet und gleichzeitig eine kurze Anlaufzeit nach einer Unterbrechung des Fertigungsprozesses ermöglicht.

### Allgemeine Beschreibung der Erfindung

Diese Aufgabe wird ausgehend von einem Betriebsverfahren der eingangs genannten Gattung erfindungsgemäß dadurch gelöst, dass bei einer Unterbrechung der kontinuierlichen Substratzuführung der UV-Strahler abgeschaltet wird, wobei die Strahler-Temperatur des abgeschalteten UV-Strahlers gemessen wird, und als Gegenmaßnahme ein Erwärmen vorgesehen ist, um einem Absinken der Strahler-Temperatur um mehr als 10 °C unter die Soll-Betriebstemperatur entgegenzuwirken.

Beim erfindungsgemäßen Betriebsverfahren wird auf einen kontinuierlichen Betrieb des UV-Strahlers und den Einsatz einer Verschlussklappe verzichtet. Stattdessen wird gemäß der Erfindung vorgeschlagen, den Strahler bei einer Unterbrechung der Substratzuführung auszuschalten. Dadurch, dass das erfindungsgemäße Betriebsverfahren auf einen kontinuierlichen Betrieb des UV-Strahlers mit Betriebsstrahlungsleistung verzichtet, wird bei einem Stillstand des Fertigungsprozesses der Energieverbrauch verringert. Hierdurch wird einerseits ein besonders energieeffizientes Betriebsverfahren ermöglicht und andererseits die Lebensdauer des Strahlers verlängert.

Der mit dem Abschirmelement verbundene Nachteil der übermäßigen Erwärmung des UV-Strahlers bei einem Stillstand des Fertigungsprozesses und die damit einhergehende Beeinträchtigung der anfänglichen Strahlungsleistung treten beim erfindungsgemäßen Verfahren nicht auf.

Um dennoch einen schnellen Start des UV-Strahlers und einen effizienten Betrieb der Vorrichtung nach einem Stillstand zu ermöglichen, werden weitere Modifikationen des Betriebsverfahrens vorgeschlagen, von denen eine die Überwachung der Strahler-Temperatur nach einem zwischenzeitlichen Abschalten des UV-Strahlers und die andere das Vorsehen von Gegenmaßnahmen betrifft, um einem Absinken der Strahler-Temperatur im ausgeschalteten Zustand entgegenzuwirken.

Der UV-Strahler ist grundsätzlich für eine vorgegebene Betriebstemperatur und eine Betriebsstrahlungsleistung ausgelegt, die bei einem optimierten Fertigungsverlauf vom UV-Strahler erreicht werden können. Dabei hat insbesondere die Betriebstemperatur des UV-Strahlers wesentlichen Einfluss auf die erzielbare Strahlungsleistung des UV-Strahlers. Sowohl eine zu hohe als auch eine zu niedrige Betriebstemperatur des UV-Strahlers gehen mit einer verringerten Strahlungsleistung einher. Eine besonders reproduzierbare Einstellung der gewünschten Strahlungsleistung wird erhalten, wenn der UV-Strahler entlang seiner Oberfläche nahezu die gleiche Temperatur aufweist.

Um auch bei einem abgeschalteten UV-Strahler einen schnellen Neustart zu ermöglichen, ist erfindungsgemäß als Gegenmaßnahme ein Erwärmen vorgesehen, um einem Absinken der Strahler-Temperatur um mehr als 10 °C unter die Soll-Betriebstem-peratur entgegenzuwirken. Hierzu wird zunächst die Strahler-Ist-Temperatur erfasst und anschließend mit der Soll-Betriebstemperatur verglichen.

Da der UV-Strahler auf einer Temperatur nahe seiner Soll-Betriebstemperatur gehalten wird, wird eine kurze Aufheizzeit ermöglicht. Dadurch, dass während des Betriebs die UV-Strahler-Temperatur höchstens um maximal 10 °C von der Betriebstemperatur abweicht, kann der UV-Strahler in weniger als 5 Sekunden seine Betriebsstrahlungsleistung erreichen.

Die Betriebsparameter der Bestrahlungsvorrichtung sind an die Betriebsstrahlungsleistung des UV-Strahlers angepasst. Im einfachsten Fall wird die Bestrahlungsvorrichtung mit einer auf die Soll-Betriebsstrahlungsleistung optimierten Zuführgeschwindigkeit betrieben. Hierdurch wird das Substrat einerseits mit ausreichender Bestrahlungsenergie bestrahlt und andererseits wird eine möglichst hohe Geschwindigkeit des Fertigungsprozesses gewährleistet.

Bei einer bevorzugten Ausgestaltung des erfindungsgemäßen Betriebsverfahrens ist vorgesehen, dass als Gegenmaßnahme ein Erwärmen des UV-Strahlers mittels eines Heizelements vorgesehen ist.

Im einfachsten Fall ist in der Nähe des UV-Strahlers eine Temperiereinheit mit einem Heizelement, beispielsweise in Form eines Infrarotstrahlers oder einer Heizwendel, vorgesehen, mit der die Strahler-Temperatur in dem Temperaturbereich um die Betriebstemperatur gehalten werden kann. Hierdurch wird es ermöglicht, dass der UV-Strahler innerhalb von wenigen Sekunden seine maximale Strahlungsleistung entfalten kann.

Bei einer alternativen, ebenso bevorzugten Ausgestaltung des erfindungsgemäßen Betriebsverfahrens ist vorgesehen, dass die Strahler-Temperatur mittels eines von einer Luftkühlung erzeugten Luftstroms beeinflusst wird, und dass als Gegenmaßnahme ein Erwärmen des Luftstroms mittels eines Heizelements vorgesehen ist.

Um den UV-Strahler mit seiner spezifischen Soll-Betriebstemperatur zu betreiben, bei der der UV-Strahler eine optimierte Strahlungsleistung aufweist, ist eine Luftkühlung für den UV-Strahler vorgesehen. Die Luftkühlung erzeugt einen Luftstrom, der an der Oberfläche des UV-Strahlers vorbeiströmt oder die Oberfläche des UV-Strahlers umströmt und so die Strahler-Temperatur in Richtung der Soll-Betriebstemperatur beeinflusst, also gegebenenfalls die aktuelle Strahler-Temperatur verringert oder erhöht. Es hat sich dabei als günstig erwiesen, wenn der Luftstrom die Oberfläche des UV-Strahlers umströmt.

Die Strahler-Temperatur kann auch durch Anpassung der Luftkühlung beeinflusst werden. In Abhängigkeit von der Temperatur der von der Luftkühlung angesaugten Umgebungsluft wird mit der Luftkühlung eine Erwärmung oder eine Kühlung der Strahleroberfläche ermöglicht; der Luftstrom kann sowohl eine Erhöhung als auch eine Verringerung der Strahler-Temperatur bewirken. Ein Luftstrom, der an dem UV-Strahler vorbeiströmt oder diesen umströmt, trägt dazu bei, dass der UV-Strahler möglichst gleichmäßig erwärmt oder gekühlt wird, und dass eine übermäßige lokale Erwärmung des UV-Strahlers vermieden wird.

Dadurch, dass das Heizelement den Luftstrom erwärmt, kann die Temperatur des UV-Strahlers durch den Luftstrom erhöht und somit im gewünschten Temperaturbereich gehalten werden. Der erwärmte Luftstrom trägt darüber hinaus zu einer gleichmäßigen Erwärmung des Strahlers bei.

Vorzugsweise ist das Heizelement ein elektrisches Heizelement mit einer stromdurchflossenen Heizwendel. Ein derartiges Heizelement ist einfach und kostengünstig zu fertigen und es weist darüber hinaus eine geringe Trägheit auf, so dass die Heizleistung vergleichsweise einfach eingestellt und angepasst werden kann. Schließlich kann ein elektrisches Heizelement einfach angesteuert werden. Vorzugsweise ist das Heizelement ein kurzwelliger Infrarotstrahler. Bei einem kurzwelligen Infrarotstrahler steht die Heizleistung sehr schnell zur Verfügung, so dass schnelle Temperaturwechsel und ein schnelles Erwärmen des UV-Strahlers ermöglicht werden.

In einer weiteren vorteilhaften Ausgestaltung des erfindungsgemäßen Betriebsverfahrens ist vorgesehen, dass die Strahler-Temperatur mittels eines von einer Luftkühlung erzeugten Luftstroms beeinflusst wird, und dass als Gegenmaßnahme ein Verändern einer Massenströmung des Luftstroms vorgesehen ist.

Dadurch, dass der Luftstrom variabel ist, wird eine Beeinflussung der StrahlerTemperatur durch eine Änderung der Massenströmung des Luftstroms ermöglicht. Ist beispielsweise die Temperatur des Luftstroms höher als die Strahler-Temperatur, wird durch eine Erhöhung der Massenströmung eine Erwärmung des Strahlers erreicht. Ist die Temperatur des Luftstroms hingegen niedriger als die StrahlerTemperatur, trägt eine Verringerung der Massenströmung dazu bei, den UV-Strahler möglichst lange warm zu halten.

Der Luftstrom der Luftkühlung ermöglicht eine exakte Einstellung der Strahler-Temperatur auch während des Betriebs der Bestrahlungsvorrichtung und trägt zu einer gleichmäßigen Strahler-Temperatur bei.

Es hat sich bewährt, wenn bei einer Unterbrechung der kontinuierlichen Substratzuführung
(aa) der UV-Strahler abgeschaltet wird, und
(bb) das Heizelement eingeschaltet wird,
   und wenn bei Aufhebung der Unterbrechung der kontinuierlichen Substratzuführung
(cc) der UV-Strahler eingeschaltet, und
(dd) das Heizelement ausgeschaltet wird.

Durch das Ausschalten des Strahlers und das Einschalten des Heizelements bei einer Unterbrechung des Produktionsprozesses wird die Strahler-Temperatur während der Unterbrechung in einem Temperaturbereich um die Betriebstemperatur gehalten. Bei einem Wiederanlaufen des Produktionsprozesses erreicht der Strahler daher unmittelbar eine hohe Strahlungsleistung. In diesem Zusammenhang hat es sich daher als günstig erwiesen, wenn der UV-Strahler eingeschaltet und gleichzeitig das Heizelement ausgeschaltet wird. Das gleichzeitige Ausschalten des Heizelements trägt dazu bei, eine übermäßige Erwärmung des UV-Strahlers unter Betriebsbedingungen zu vermeiden.

Es hat sich als günstig erwiesen, wenn das Erwärmen des Luftstroms in einem Luftzuführungskanal der Luftkühlung erfolgt.

Ein in einem Luftzuführungskanal angeordnetes Heizelement hat den Vorteil, dass die Luft in räumlicher Nähe zum UV-Strahler erwärmt werden kann, so dass ein besonders energieeffizientes Betriebsverfahren ermöglicht wird. Gleichzeitig wird einer ungleichmäßigen Erwärmung des UV-Strahlers entgegengewirkt.

Bei einer bevorzugten Ausgestaltung des erfindungsgemäßen Verfahrens ist vorgesehen, dass die Bestrahlungsvorrichtung einen Reflektor mit einer dem UV-Strahler zugewandten und einer dem UV-Strahler abgewandten Seite aufweist, und dass das Erwärmen des Luftstroms durch ein auf der abgewandten Seite des Reflektors angeordnetes Heizelement erfolgt.

Der Reflektor ist mit dem UV-Strahler fest verbunden oder es handelt sich um ein separat davon angeordnetes Reflektorbauteil; er weist eine dem UV-Strahler zugewandte und eine dem UV-Strahler abgewandte Seite auf.

Dadurch, dass das Heizelement hinter dem Reflektor, also auf der dem UV-Strahler abgewandten Seite angeordnet ist, wird von diesem unmittelbar nur der Reflektor erwärmt. Dadurch, dass der UV-Strahler keiner direkten Erwärmung durch das Heizelement ausgesetzt ist und allenfalls mittelbar über den Reflektor erwärmt wird, wird eine ungleichmäßige und lokale Erwärmung des UV-Strahlers vermieden. Eine solche Anordnung trägt daher zu einer gleichmäßigen Erwärmung des UV-Strahlers bei.

Vorzugsweise umströmt der Luftstrom den UV-Strahler in einer Richtung senkrecht zur Strahler-Längsrichtung.

Hierdurch wird eine gleichmäßige Temperierung des UV-Strahlers ermöglicht.

Es hat sich bewährt, wenn die Zuführgeschwindigkeit fortlaufend von einem Sensor erfasst wird.

Eine wirkungsvolle Anpassung der Strahlungsleistung des UV-Strahlers an die Zuführgeschwindigkeit wird ermöglicht, wenn die Zuführgeschwindigkeit fortlaufend - das heißt kontinuierlich oder von Zeit zu Zeit - ermittelt wird. Der zur Ermittlung der Zuführgeschwindigkeit vorgesehene Sensor kann die Zuführgeschwindigkeit beispielsweise durch Erfassung einer elektrischen oder optischen Messgröße erfassen. Vorzugsweise erfolgt die Messung der Zuführgeschwindigkeit berührungslos unter Einsatz eines optischen Korrelationsmesssystems, beispielsweise mittels einer Kamera.

Es hat sich als günstig erwiesen, wenn die Temperatur des UV-Strahlers fortlaufend von einem Sensor erfasst wird.

Der Temperatursensor wandelt die Temperatur in eine elektrische Messgröße um. Er erfasst die Temperatur des UV-Strahlers fortlaufend, das heißt kontinuierlich oder von Zeit zu Zeit. Insbesondere bei gleichzeitigem Einsatz mehrerer UV-Strahler kann jeder der Strahler mit einem Temperatursensor versehen sein. Alternativ kann auch nur die Temperatur an einem einzigen Strahler oder an einzelnen Strahlern erfasst werden. Die Erfassung der Temperatur erfolgt vorzugsweise an der Oberfläche des Strahlerrohrs. Durch die fortlaufende Erfassung der Strahler-Temperatur ist es möglich, Abweichungen der Strahler-Temperatur von einem vorgegebenen Soll-Wert möglichst schnell zu erkennen. Hierdurch wird ein besonders dynamisches Betriebsverfahren gewährleistet.

### Ausführungsbeispiel

Nachfolgend wird die Erfindung anhand eines Ausführungsbeispiels und mehreren Zeichnungen näher beschrieben. Dabei zeigt in schematischer Darstellung:
- **Figur 1**: eine Ausführungsform einer nach dem erfindungsgemäßen Betriebsverfahren arbeitenden Bestrahlungsvorrichtung zur Bestrahlung eines Substrats,
- **Figur 2**: ein erstes Strahlermodul zum Einsatz in der Bestrahlungsvorrichtung gemäß Figur 1, bei dem in einem Luftzuführungskanal eine Heizwendel angeordnet ist,
- **Figur 3**: das Strahlermodul gemäß Figur 2 in einer Rückansicht,
- **Figur 4**: ein zweites Strahlermodul zum Einsatz in der Bestrahlungsvorrichtung gemäß Figur 1, bei dem ein senkrecht zur Längsrichtung des Strahlermoduls verlaufendes Heizelement hinter einem Reflektor angeordnet ist,
- **Figur 5**: ein drittes Strahlermodul zum Einsatz in der Bestrahlungsvorrichtung gemäß Figur 1, bei dem hinter einem Reflektor ein in Längsrichtung des Strahlermoduls verlaufendes Heizelement angeordnet ist, und
- **Figur 6**: ein Diagramm, in dem die relative UV-Emission eines nach dem erfindungsgemäßen Verfahren betriebenen UV-Strahlers in Abhängigkeit von der Zeit nach dem Start des Strahlers für unterschiedlich stark vortemperierte Strahler dargestellt ist.

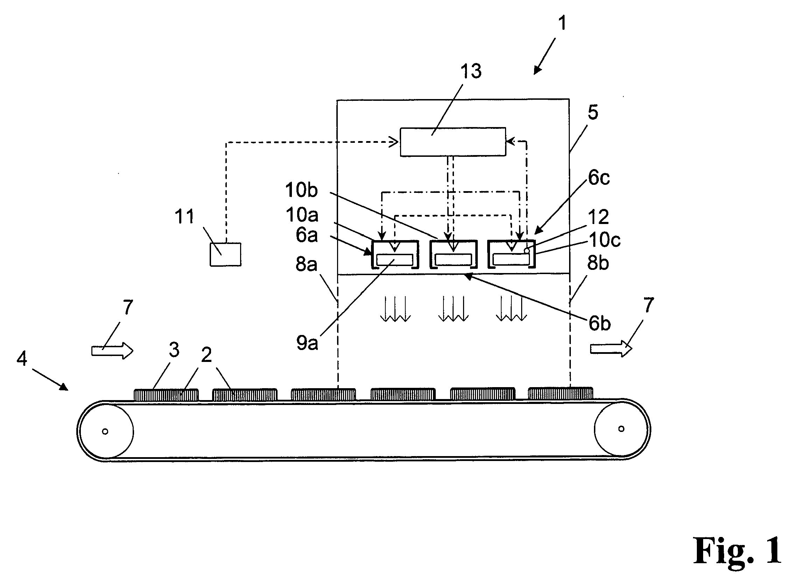

**Figur 1** zeigt schematisch eine Ausführungsform einer nach dem erfindungsgemäßen Betriebsverfahren arbeitenden Bestrahlungsvorrichtung, der insgesamt die Bezugsziffer 1 zugeordnet ist. Die Bestrahlungsvorrichtung 1 wird zum Vernetzen und Härten einer Beschichtung 3 auf Werkstücken 2 in Form von Kunststoff-Folien eingesetzt.

Die Bestrahlungsvorrichtung 1 umfasst eine Strahlereinheit 5 zur Bestrahlung der Werkstücke 2 und eine Transportvorrichtung 4, die die Werkstücke 2 in Transportrichtung 7 der Bestrahlung durch die Strahlereinheit 5 kontinuierlich zuführt.

Die Strahlereinheit 5 weist drei hintereinander angeordnete Strahlermodule 6a, 6b, 6c, sowie eine Regeleinheit 13 für die Strahlermodule 6a, 6b, 6c auf. Die Strahlermodule 6a, 6b, 6c sind identisch ausgebildet. Nachfolgend ist daher nur das Strahlermodul 6a näher beschrieben.

Das Strahlermodul 6a umfasst einen UV-Strahler 9a, dem ein Heizelement 10a zur Erwärmung des UV-Strahlers 9a zugeordnet ist. Der UV-Strahler 9a weist ein zylinderförmiges Strahlerrohr aus Quarzglas mit einer Strahlerrohr-Längsachse auf. Er zeichnet sich durch eine Nominal-Leistung von 300 W und eine Länge des Strahlerrohrs von 1.000 mm aus.

Die Strahlermodule 6a, 6b, 6c sind innerhalb der Strahlereinheit 5 relativ zur Transportvorrichtung 4 derart angeordnet, dass die Strahlerrohrlängsachsen der UV-Strahler senkrecht zur Transportrichtung 7 verlaufen. Die Strahlereinheit 5 legt auf der Oberfläche der Transportvorrichtung 4 ein Bestrahlungsfeld für die Bestrahlung der Werkstücke 2 fest. Die Erstreckung des Bestrahlungsfelds in Transportrichtung 7 ist in Figur 1 durch gestrichelte Linien 8a, 8b eingezeichnet.

Die Transportvorrichtung 4 bewegt die Werkstücke 2 relativ zur Strahlereinheit 5, so dass diese das Bestrahlungsfeld langsam durchlaufen. Der Abstand der Strahlereinheit 5 zur Oberfläche des Werkstücks 2 beträgt 20 mm und kann über eine Vorrichtung zur Einstellung des Abstands (nicht dargestellt) eingestellt werden.

Der Bestrahlungsvorrichtung 1 liegt das erfindungsgemäße Betriebsverfahren zugrunde. Bevor die Werkstücke 2 dem Bestrahlungsfeld der Strahlereinheit 5 zugeführt werden, werden zunächst die UV-Strahler 9a, 9b, 9c eingeschaltet, so dass diese ihre Betriebstemperatur erreichen. In einer alternativen Ausgestaltung des Betriebsverfahrens ist vorgesehen, dass die UV-Strahler zunächst jeweils durch das zugehörige Heizelement 10a, 10b, 10c vorgewärmt beziehungsweise dauerhaft auf Betriebstemperatur gehalten und anschließend gestartet werden.

Haben die UV-Strahler 9a, 9b, 9c ihre vorgegebene Betriebstemperatur und Betriebsstrahlungsleistung erreicht, werden die Werkstücke 2 von der Transportvorrichtung 4 mit vorgegebener Transportgeschwindigkeit dem Bestrahlungsbereich zugeführt. Um einen effizienten Betrieb der Bestrahlungsvorrichtung 1 zu ermöglichen, ist die Transportgeschwindigkeit an die mittlere Betriebsstrahlungsleistung der UV-Strahler 9a, 9b, 9c angepasst. Die Werkstücke 2 durchlaufen den Bestrahlungsbereich dabei mit möglichst konstanter Transportgeschwindigkeit. Die Transportgeschwindigkeit wird kontinuierlich über einen optischen Sensor 11 erfasst, der den in einem vorgegebenen Zeitintervall zurückgelegten Weg eines Werkstücks 2 ermittelt. Der Sensor 11 übermittelt die Transportgeschwindigkeit kontinuierlich an die Regeleinheit 13 der Strahlereinheit 5.

Kommt es während des Prozesses zu einem Stillstand des Produktionsprozesses, besteht die Gefahr, dass die im Bestrahlungsbereich befindlichen Werkstücke 2 zulange einer UV-Bestrahlung ausgesetzt sind, so dass diese beschädigt werden können. Um dies zu vermeiden, ist vorgesehen, dass die Betriebsparameter der Strahlermodule 6a, 6b, 6c durch die Regeleinheit 13 in Abhängigkeit von der Transportgeschwindigkeit geregelt werden. Bei einem Stillstand der Produktion werden die Strahlermodule 6a, 6b, 6c ausgeschaltet.

Um bei einem Wiederanlaufen der Produktion möglichst unmittelbar eine Bestrahlung der Werkstücke 2 mit einer hohen Bestrahlungsleistung gewährleisten zu können, wird gleichzeitig die Temperatur der UV-Strahler 9a, 9b, 9c gemessen. Zur Erfassung der Strahler-Temperatur ist auf dem Strahlerrohr des UV-Strahlers 9c von Strahlermodul 6c ein Temperatursensor 12 angeordnet, der die IstTemperatur des Strahlerrohrs erfasst. In einer alternativen Ausführungsform (nicht dargestellt) ist jedes Strahlermodul 6a, 6b, 6c mit einem Temperatursensor 12 versehen. Sinkt die Temperatur des UV-Strahlers 9a, 9b, 9c um mehr als 10 °C unter dessen Betriebstemperatur ab, wird von der Regeleinheit 13 das jeweilige Heizelement 10a, 10b, 10c eingeschaltet, so dass der Luftstrom mit dem der UV-Strahler in einer Richtung senkrecht zur Strahler-Längsrichtung umspült wird, erwärmt wird. Die UV-Strahler 9a, 9b, 9c werden so während des Stillstands der Produktion auf einer Temperatur im Bereich ihrer Betriebstemperatur gehalten.

Dadurch, dass der UV-Strahler 9a, 9b, 9c auf Betriebstemperatur gehalten wird, wird die Zeit verringert, die der UV-Strahler 9a, 9b, 9c bei einem erneuten Start benötigt, um seine Betriebsstrahlungsleistung zu erreichen. Hierdurch wird ein unmittelbarer Start der Bestrahlungsvorrichtung 1 nach einem Stillstand mit hoher Transportgeschwindigkeit ermöglicht. Bei einem Wiederanlaufen der Produktion wird gleichzeitig mit dem erneuten Einschalten des UV-Strahlers 9a, 9b, 9c das Heizfilament 10a, 10b, 10c ausgeschaltet.

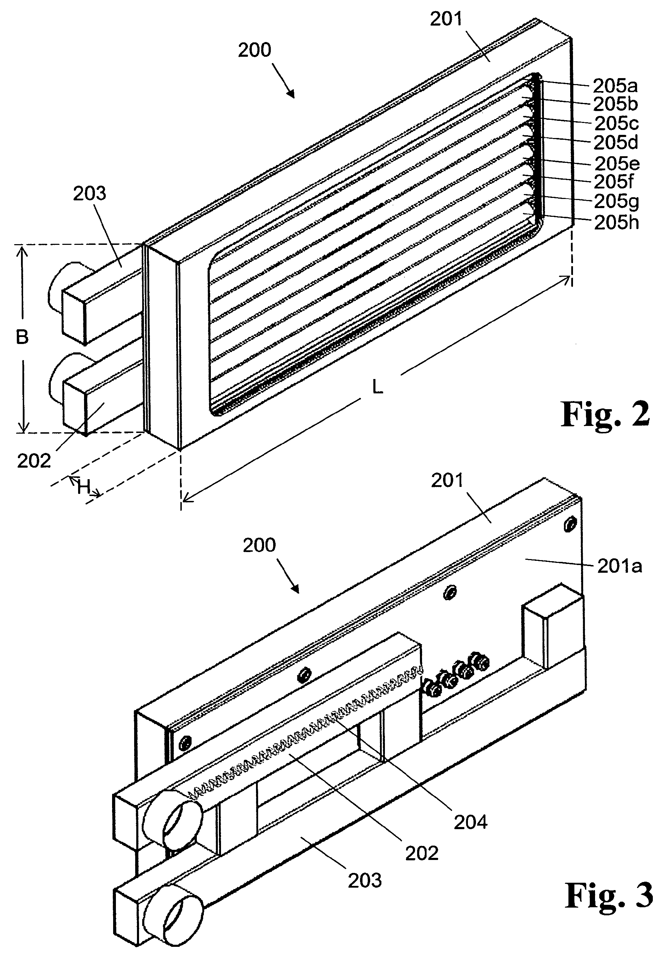

**Figur 2** zeigt schematisch eine Vorderansicht eines Strahlermoduls 200, das in die Bestrahlungsvorrichtung gemäß Figur 1 eingesetzt werden kann.

Das Strahlermodul 200 umfasst ein Gehäuse 201 mit acht darin angeordneten UV-Strahlern 205a-205h. Das Gehäuse 201 ist aus Edelstahl gefertigt. Es weist eine Länge L von 1.030 mm, eine Breite B von 434 mm und eine Höhe H von 171 mm auf. Auf der Rückseite des Gehäuses 201 sind Lüftungskanäle 202, 203 angeordnet.

Die UV-Strahler 205a-205h weisen jeweils ein an beiden Enden verschlossenes, zylinderförmiges Strahlerrohr aus Quarzglas mit einer Strahlerrohr-Längsachse auf. Die UV-Strahler 205a-205h zeichnen sich durch eine Nominal-Leistung von 300 W (bei einem nominalen Lampenstrom von 4 A), eine Strahlerrohr-Länge von 100 cm, einen Strahlerrohr-Außendurchmesser von 28 mm und durch eine Leistungsdichte von 3 W/cm aus; sie sind innerhalb des Gehäuses derart angeordnet, dass ihre Strahlerrohr-Längsachsen parallel zueinander verlaufen.

**Figur 3** zeigt schematisch eine Rückansicht des Strahlermodul 200 zum Einsatz in der Bestrahlungsvorrichtung gemäß Figur 1. Das Strahlermodul 200 umfasst ein Gehäuse 201 mit acht darin angeordneten UV-Strahlern 205a-205h (in der Zeichnung nicht ersichtlich). Auf der Rückseite 201a des Gehäuses 201 sind Lüftungskanäle 202, 203 angeordnet, durch die die UV-Strahler während des Betriebs über einen Luftstrom gekühlt werden können, der die Strahler in einer Richtung senkrecht zur Strahler-Längsrichtung anströmt. Der Lüftungskanal 202 ist ein Zuluftkanal, der Lüftungskanal 203 wird als Abluftkanal verwendet. Im Lüftungskanal 202 ist eine Heizwendel 204 angeordnet.

Wird das Strahlermodul 200 mit Nominalleistung betrieben, kommt es zu einer Erwärmung der in das Strahlermodul 200 eingebauten UV-Strahler 205a-205h. Um eine übermäßige Erwärmung der UV-Strahler 205a-205h und des Gehäuses 201 zu vermeiden und um die UV-Strahler 205a-205h mit optimierter Strahlungsleistung betreiben zu können, können die Strahler 205a-205h über den Lüftungskanal 202 mit einem Kühlluftstrom umspült und dadurch gekühlt werden. Die von den Strahlern 205a-205h erwärmte Kühlluft wird dabei durch den Abluftkanal 203 abgeführt. Der Luftstrom ist variabel; insbesondere kann zur Anpassung der Kühlleistung die Massenströmung des Luftstroms angepasst werden.

Um eine Abkühlung der ausgeschalteten UV-Strahler 205a-205h zu vermeiden, ist im Zuluftkanal 202 ein Heizelement 204 angeordnet, das bei Bedarf eingeschaltet werden kann. Das Heizelement 204 dient der Erwärmung der durch den Zuluftkanal 202 zugeführten Luft, die wiederum zu einer Erwärmung der UV-Strahler 205a-205h beiträgt. Durch eine Regelung der Zuluft-Temperatur können die UV-Strahler 205a-205h auf Betriebstemperatur gehalten werden.

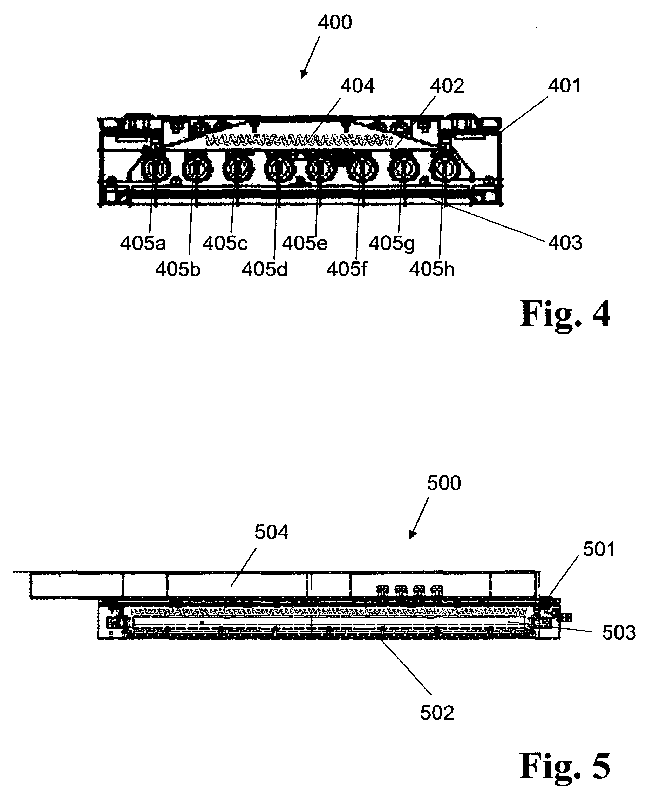

In **Figur 4** ist schematisch in einer Querschnittsdarstellung eine zweite Ausführungsform eines Strahlermoduls zum Einsatz in der Bestrahlungsvorrichtung gemäß Figur 1 dargestellt. Dem Strahlermodul ist insgesamt die Bezugsziffer 400 zugeordnet. Die Bemaßung des Strahlermoduls 400 ist in Figur 1 in mm angegeben. Das Strahlermodul 400 umfasst ein Gehäuse 401 mit acht darin angeordneten UV-Strahler 405a-405h und ein Gehäusefenster 403 aus Quarzglas. Darüber hinaus ist auf der Innenseite des Strahlermoduls 400 ein Reflektor 402 aus Aluminium angebracht. Im Gegensatz zum Strahlungsmodul 200 aus Figur 2 und 3 weist das Strahlermodul 400 keine Lüftkühlung auf. Darüber hinaus ist hinter dem Reflektor 402 ein Heizelement 404 angeordnet, das den Reflektor 402 und damit mittelbar auch die UV-Strahler 405a-405h erwärmt. Das Heizelement 404 verläuft dabei senkrecht zur Längsachse des Strahlermoduls 400. In Richtung der Längsachse gesehen sind vier parallel zueinander verlaufende Heizelemente (nicht dargestellt) angeordnet.

**Figur 5** zeigt schematisch eine dritte Ausführungsform eines Strahlermoduls, dem insgesamt die Bezugsziffer 500 zugeordnet ist. Das Strahlermodul 500 umfasst ein Gehäuse 501 mit vier darin angeordneten UV-Strahlern 503, auf dessen Rückseite ein Luftkühlsystem 504 zur Kühlung der UV-Strahler 503 angebracht ist. In die Vorderseite des Gehäuses 501 ist ein für ultraviolette Strahlung durchlässiges Fenster 502 aus Quarzglas eingelassen. Zwischen der rückseitigen Wand des Gehäuses 501 und den UV-Strahlern 503 ist ein Heizelement angeordnet, das parallel zur Längsachse der UV-Strahler 503 verläuft.

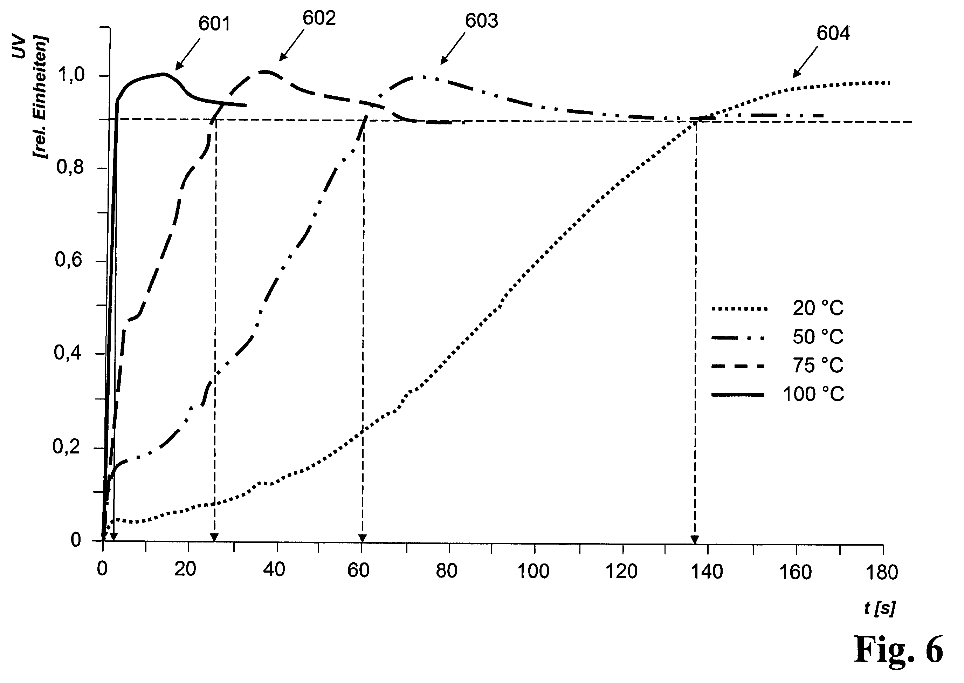

Das Diagramm in **Figur 6** zeigt die UV-Emission eines UV-Strahlers bei der Wellenlänge 254 nm in Abhängigkeit von der Zeit nach dem Start des UV-Strahlers für verschiedene Strahler-Starttemperaturen.

Als UV-Strahler wurde ein Niederdruckstrahler mit einem Strahlerrohr aus Quarzglas verwendet, das an beiden Enden über Quetschungen verschlossen ist. Das Strahlerrohr des Niederdruckstrahlers umschließt einen mit Argon gefüllten Entladungsraum, in dem ein Amalgamdepot und zwei Elektroden angeordnet sind.

Der Niederdruckstrahler zeichnet sich durch eine Nominal-Leistung von 300 W (bei einem nominalen Lampenstrom von 4 A), eine Strahlerrohr-Länge von 100 cm, einen Strahlerrohr-Außendurchmesser von 28 mm und durch eine Leistungsdichte von 3 W/cm aus.

Der Niederdruckstrahler wurde vor dem Start zunächst auf eine Starttemperatur erwärmt. Hierzu wurde die Temperatur des Niederdruckstrahlers in der Mitte des Strahlerrohrs mit einem auf der Außenseite des Strahlerrohrs angebrachten Temperatursensor bestimmt. Als Starttemperaturen wurden 20 °C, 50 °C, 75 °C und 100°C gewählt. Anschließend wurde der Niederdruckstrahler zum Zeitpunkt t=0 gestartet. In Figur 6 ist für jede dieser Starttemperaturen ein Verlauf der UV-Emission in Abhängigkeit von der Zeit nach dem Start dargestellt. Auf der Abszisse ist die Zeit seit dem Start des Strahlers in Sekunden aufgetragen. Die Ordinate gibt die ultraviolette Strahlungsemission in relativen Einheiten wieder.

Für eine gute UV-Emissionsleistung muss der Niederdruckstrahler eine gewisse Temperatur aufweisen. Da der Niederdruckstrahler sich während des Betriebs erwärmt, stellt sich diese nach einer gewissen Betriebszeit ein. Wie der Kurvenverlauf 604 zeigt, stellt sich bei einem Strahler, der auf eine Temperatur von 20°C vorerwärmt wurde, eine akzeptable UV-Emission nach etwa 135 s ein. Die Zeit bis zum Erreichen einer akzeptablen UV-Emission kann durch ein Vorwärmen des Strahlerrohrs erreicht werden. Eine Starttemperatur von 50 °C führt gemäß Kurvenverlauf 603 zu einer Startzeit von etwa 65 s. Bei einer Starttemperatur von 75 °C verkürzt sich die Startzeit auf etwa 23 s und insbesondere bei einer Starttemperatur von 100 °C kann eine Startzeit von weniger als 5 s erreicht werden (Kurvenverläufe 601, 602).

**Bezugszeichenliste**

| | |
|---|---|
| Bestrahlungsvorrichtung | 1 |
| Werkstücke | 2 |
| Beschichtung | 3 |
| Transportvorrichtung | 4 |
| Strahlereinheit | 5 |
| Strahlermodule | 6a, 6b, 6c |
| Transportrichtung | 7 |
| Bestrahlungsfeld (Linien) | 8a, 8b |
| UV-Strahler | 9a |
| Heizelemente | 10a, 10b, 10c |
| Optischer Sensor | 11 |
| Temperatursensor | 12 |
| Regeleinheit | 13 |
| Strahlermodul | 200 |
| Gehäuse | 201 |
| Gehäuse-Rückseite | 201 a |
| Lüftungskanäle | 202,203 |
| Heizwendel | 204 |
| UV-Strahler | 205a-205h |
| Strahlermodul | 400 |
| Gehäuse | 401 |
| Reflektor | 402 |
| Gehäusefenster | 403 |
| Heizelement | 404 |
| UV-Strahler | 405a-405h |
| Strahlermodul | 500 |
| Gehäuse | 501 |
| Fenster | 502 |
| UV-Strahler | 503 |
| Luftkühlsystem | 504 |
| Kurvenverläufe | 601-604 |

## Patentansprüche

1. Betriebsverfahren für eine Bestrahlungsvorrichtung zur Bestrahlung eines Substrats (2) mit einem UV-Strahler(9a; 205a-205h; 405a-405h, 503), umfassend die Verfahrensschritte:
(a) Betreiben des UV-Strahlers (9a; 205a-205h; 405a-405h, 503) mit einer von einer Soll-Betriebstemperatur abhängigen Soll-Betriebsstrahlungsleistung,
(b) kontinuierliches Zuführen des Substrats (2) in einen durch den UV-Strahler (9a; 205a-205h; 405a-405h, 503) festgelegten Bestrahlungsbereich mit einer Zuführgeschwindigkeit,
(c) Bestrahlen des Substrats (2) im Bestrahlungsbereich,
**dadurch gekennzeichnet, dass** bei einer Unterbrechung der kontinuierlichen Substratzuführung der UV-Strahler (9a; 205a-205h; 405a-405h, 503) abgeschaltet wird, wobei die Strahler-Temperatur des abgeschalteten UV-Strahlers (9a; 205a-205h; 405a-405h, 503) gemessen wird, und als Gegenmaßnahme ein Erwärmen vorgesehen ist, um einem Absinken der Strahler-Temperatur um mehr als 10 °C unter die Soll-Betriebstemperatur entgegenzuwirken.

2. Betriebsverfahren nach Anspruch 1, **dadurch gekennzeichnet, dass** als Gegenmaßnahme das Erwärmen des UV-Strahlers (9a; 205a-205h; 405a-405h) mittels eines Heizelements (10a, 10b, 10c, 404) vorgesehen ist.

3. Betriebsverfahren nach Anspruch 1, dass die Strahler-Temperatur mittels eines von einer Luftkühlung (202; 203) erzeugten Luftstroms beeinflusst wird, und dass als Gegenmaßnahme das Erwärmen des Luftstroms mittels eines Heizelements (204) vorgesehen ist.

4. Betriebsverfahren nach einem der vorhergehenden Ansprüche, **dadurch gekennzeichnet, dass** die Strahler-Temperatur mittels eines von einer Luftkühlung (202; 203) erzeugten Luftstroms beeinflusst wird, und dass als Gegenmaßnahme ein Verändern einer Massenströmung des Luftstroms vorgesehen ist.

5. Betriebsverfahren nach Anspruch 2 oder 3, **dadurch gekennzeichnet, dass** bei einer Unterbrechung der kontinuierlichen Substratzuführung
(aa) der UV-Strahler (9a; 205a-205h; 405a-405h) abgeschaltet wird, und
(bb) das Heizelement (10a, 10b, 10c, 404) eingeschaltet wird,
und dass bei Aufhebung der Unterbrechung der kontinuierlichen Substratzuführung
(cc) der UV-Strahler (9a; 205a-205h; 405a-405h, 503) eingeschaltet, und
(dd) das Heizelement (10a, 10b, 10c, 404) ausgeschaltet wird.

6. Betriebsverfahren nach Anspruch 3, **dadurch gekennzeichnet, dass** das Erwärmen des Luftstroms in einem Luftzuführungskanal (202) der Luftkühlung (202; 203) erfolgt.

7. Betriebsverfahren nach einem der vorhergehenden Ansprüche 3 oder 6, **dadurch gekennzeichnet, dass** die Bestrahlungsvorrichtung einen Reflektor (402) mit einer dem UV-Strahler (405a-405h) zugewandten und einer dem UV-Strahler (405a-405h) abgewandten Seite aufweist, und dass das Erwärmen des Luftstroms durch ein auf der abgewandten Seite des Reflektors (402) angeordnetes Heizelement (404) erfolgt.

8. Betriebsverfahren nach einem der vorhergehenden Ansprüche 3, 4, 6 oder 7, **dadurch gekennzeichnet, dass** der Luftstrom den UV-Strahler (9a; 205a-205h) in einer Richtung senkrecht zur Strahler-Längsrichtung umströmt.

9. Betriebsverfahren nach einem der vorhergehenden Ansprüche, **dadurch gekennzeichnet, dass** die Zuführgeschwindigkeit fortlaufend von einem Sensor (11) erfasst wird.

10. Betriebsverfahren nach einem der vorhergehenden Ansprüche, **dadurch gekennzeichnet, dass** die Temperatur des UV-Strahlers (9a; 205a-205h; 405a-405h, 503) fortlaufend von einem Sensor (12) erfasst wird.

## Claims

1. Operating process for an irradiation device for irradiating a substrate (2) by means of an UV emitter (9a; 205a-205h; 405a-405h, 503), comprising the process steps of:
(a) Operating the UV emitter (9a; 205a-205h; 405a-405h, 503) at a nominal operating radiation power that is a function of a nominal operating temperature;
(b) continuously feeding the substrate (2), at a feed rate, into an irradiation area that is defined by the UV emitter (9a; 205a-205h; 405a-405h, 503);
(c) irradiating the substrate (2) in the irradiation area;
**characterised in that** the UV emitter (9a; 205a-205h; 405a-405h, 503) is switched off if there is an interruption of the continuous substrate feed, whereby the emitter temperature of the switched-off UV emitter (9a; 205a-205h; 405a-405h, 503) is measured and provisions for heating are made as a counter-measure to counteract a decrease of the emitter temperature by more than 10 °C below the nominal operating temperature.

2. Operating process according to claim 1, **characterised in that** it provides for heating of the UV emitter (9a; 205a-205h; 405a-405h) by means of a heating element (10a, 10b, 10c, 404) as a counter-measure.

3. Operating process according to claim 1, **characterised in that** the emitter temperature is influenced by means of an air flow generated by an air cooling (202; 203) and **in that** heating of the air flow by means of a heating element (204) is provided as counter-measure.

4. Operating process according to any one of the preceding claims, **characterised in that** the emitter temperature is influenced by means of an air flow generated by an air cooling (202; 203) and **in that** changing a mass flow of the air flow is provided as counter-measure.

5. Operating process according to claim 2 or 3, **characterised in that**, if the continuous substrate feed is interrupted,
(aa) the UV emitter (9a; 205a-205h; 405a-405h) is switched off; and
(bb) the heating element (10a, 10b, 10c, 404) is switched on;
and when the interruption of the continuous substrate feed is no longer present;
(cc) the UV emitter (9a; 205a-205h; 405a-405h, 503) is switched on; and
(dd) the heating element (10a, 10b, 10c, 404) is switched off.

6. Operating process according to claim 3, **characterised in that** the heating of the air flow takes place in an air feed channel (202) of the air cooling (202; 203).

7. Operating process according to either one of the preceding claims 3 or 6, **characterised in that** the irradiation device comprises a reflector (402) having a side facing the UV emitter (405a-405h) and a side facing away from the UV emitter (405a-405h), and **in that** the air flow is heated by a heating element (404) that is arranged on the side of the reflector (402) facing away.

8. Operating process according to any one of the preceding claims 3, 4, 6 or 7, **characterised in that** the air flow flows around the UV emitter (9a; 205a-205h) in a direction perpendicular to the longitudinal direction of the emitter.

9. Operating process according to any one of the preceding claims, **characterised in that** the feed rate is detected consecutively by a sensor (11).

10. Operating process according to any one of the preceding claims, **characterised in that** the temperature of the UV emitter (9a; 205a-205h; 405a-405h, 503) is detected consecutively by a sensor (12).

## Revendications

1. Procédé de fonctionnement pour un dispositif d'irradiation pour irradier un substrat (2) comprenant un émetteur UV (9a ; 205a-205h ; 405a-405h, 503), comprenant les étapes de procédé suivantes :
(a) fonctionnement de l'émetteur UV (9a ; 205a-205h ; 405a-405h, 503) à une puissance d'irradiation de consigne qui est fonction d'une température de fonctionnement de consigne,
(b) alimentation en continu du substrat (2) dans une zone d'irradiation déterminée par l'émetteur UV (9a ; 205a-205h ; 405a-405h, 503), à une vitesse d'alimentation,
(c) irradiation du substrat (2) dans la plage d'irradiation,
**caractérisé en ce que** lors d'une interruption de l'alimentation en continu du substrat, l'émetteur UV (9a ; 205a-205h ; 405a-405h, 503) est désactivé, dans lequel la température d'émetteur de l'émetteur UV (9a ; 205a-205h ; 405a-405h, 503) désactivé est mesurée et en contre-mesure, un réchauffement est prévu pour contrer une baisse de la température de l'émetteur de plus de 10 °C en-dessous de la température de fonctionnement de consigne.

2. Procédé de fonctionnement selon la revendication 1, **caractérisé en ce que** le réchauffement de l'émetteur UV (9a ; 205a-205h ; 405a- 405h) est prévu au moyen d'un élément de chauffage (10a, 10b, 10c, 404) en contre-mesure.

3. Procédé de fonctionnement selon la revendication 1, **caractérisé en ce que** la température d'émetteur est influencée au moyen d'un courant d'air produit par un refroidissement d'air (202 ; 203) et qu' en contre-mesure, le réchauffement du courant d'air est prévu au moyen d'un élément de chauffage (204).

4. Procédé de fonctionnement selon l'une des revendications précédentes, **caractérisé en ce que** la température d'émetteur est influencée au moyen d'un courant d'air produit par un refroidissement d'air (202 ; 203) et qu'une modification du débit massique du courant est prévue en contre-mesure.

5. Procédé de fonctionnement selon la revendication 2 ou 3, **caractérisé en ce que** lors d'une interruption de l'alimentation en continu du substrat
(aa) l'émetteur UV (9a ; 205a-205h ; 405a-405h) est désactivé, et
(bb) l'élément de chauffage (10a, 10b, 10c, 404) est activé,
et que lors de l'arrêt de l'interruption de l'alimentation en continu du substrat
(cc) l'émetteur UV (9a ; 205a-205h ; 405a-405h) est activé, et
(dd) l'élément de chauffage (10a, 10b, 10c, 404) est désactivé.

6. Procédé de fonctionnement selon la revendication 3, **caractérisé en ce que** le réchauffement du courant d'air a lieu dans un canal d'amenée d'air (202) du refroidissement d'air (202 ; 203).

7. Procédé de fonctionnement selon l'une des revendications précédentes 3 ou 6, **caractérisé en ce que** le dispositif d'irradiation présente un réflecteur (402) avec un côté tourné vers l'émetteur UV (405a-405h) et un côté détourné de l'émetteur UV (405a-405h), et que le réchauffement du courant d'air s'effectue par un élément de chauffage (404) disposé sur le côté détourné du réflecteur (402).

8. Procédé de fonctionnement selon l'une des revendications précédentes 3, 4, 6 ou 7, **caractérisé en ce que** le courant d'air enveloppe l'émetteur UV (9a ; 205a- 205h) dans un sens vertical à un sens longitudinal de l'émetteur.

9. Procédé de fonctionnement selon l'une des revendications précédentes, **caractérisé en ce que** la vitesse d'alimentation est détectée en continu par un capteur (11).

10. Procédé de fonctionnement selon l'une des revendications précédentes, **caractérisé en ce que** la température de l'émetteur UV (9a ; 205a-205h ; 405a-405h, 503) est détectée en continu par un capteur (12).
